# EUROPÄISCHE PATENTSCHRIFT

(11) **EP 0 910 629 B1**
(45) Veröffentlichungstag und Bekanntmachung des Hinweises auf die Patenterteilung: **28.07.2004**
(21) Anmeldenummer: 97927036.0
(22) Anmeldetag: 26.05.1997
(51) Int. Cl.: C12N 9/74, A61K 38/48

(54) **Verfahren zur Herstellung einer Antidotausgangssubstanz für natürliche und synthetische Thrombininhibitoren**
PROCESS FOR PRODUCING A BASIC ANTIDOTE SUBSTANCE FOR NATURAL AND SYNTHETIC THROMBIN INHIBITORS
PROCEDE DE PREPARATION D'UNE SUBSTANCE DE BASE ANTIDOTE POUR DES INHIBITEURS NATURELS ET SYNTHETIQUES DE LA THROMBINE

(30) Priorität: 26.06.1996 DE 19625642
(43) Veröffentlichungstag der Anmeldung: 28.04.1999
(73) Patentinhaber: Max-Planck-Gesellschaft zur Förderung der Wissenschaften e.V. Berlin, 80539 München (DE)
(72) Erfinder: NOWAK, Götz, D-99097 Erfurt (DE); ZABE, Martin, D-07747 Jena (DE); BUCHA, Elke, D-99094 Erfurt (DE)
(74) Vertreter: Albrecht, Thomas, Dr.
(86) Internationale Anmeldenummer: PCT/EP1997/002678
(87) Internationale Veröffentlichungsnummer: WO 1997/049801

(56) Entgegenhaltungen:
- EP-A- 0 617 049
- WO-A-93/15757
- DE-A- 4 430 204
- REEKERS P ET AL: "PURIFICATION OF BLOOD COAGULATION FACTORS II, VII, IX AND X FROM BOVINE CITRATED PLASMA" NEUROBIOLOGY OF AGING, Bd. 1, Nr. 1, Seiten 2-22, XP000602120
- ZABE M ET AL: "Characterization of human meizothrombin and meizothrombin (DESF1) produced with immobilized ecarin." 39TH ANNUAL MEETING OF THE SOCIETY FOR THROMBOSIS AND HEMOSTASIS RESEARCH, BERLIN, GERMANY, FEBRUARY 15-18, 1995. ANNALS OF HEMATOLOGY 70 (SUPPL.). 1995. A87. ISSN: 0939-5555, XP002042628
- WEINSTEIN, RALPH E. ET AL: "Purification and preliminary characterization of rabbit vitamin K-dependent coagulation proteins" THROMB. RES. (1990), 59(4), 759-72 CODEN: THBRAA;ISSN: 0049-3848, 1990, XP002042629
- S.P. BAJAJ ET AL: "A simplified procedure for purification of human prothrombin, factor IX and factor X" PREPARATIVE BIOCHEMSITRY, Bd. 11, Nr. 4, 1981, Seiten 397-412, XP002042630

## Beschreibung

Die Erfindung betrifft ein Verfahren zur Herstellung einer hochgereinigten virusfreien Antidotausgangssubstanz für natürliche und synthetische Thrombininhibitoren sowie die Verwendung einer so erhaltenen Antidotausgangssubstanz zur Herstellung eines Antidots für einen natürlichen oder synthetischen Thrombininhibitor. Bevorzugt ist die Antidotausgangssubstanz Prothrombin oder Präthrombin-1.

Antikoagulantien, wie beispielsweise Heparin oder Cumarine, finden heute zur Therapie und Prophylaxe thromboembolischer Erkrankungen, insbesondere zur Behandlung des Herzinfarkts, der Arteriosklerose, ferner bei Bluttransfusionen und nach Operationen vielfach Verwendung.

Hirudin, das aus der Speicheldrüse von Hirudo medicinalis (Blutegel) gewonnen wird, ist ein Antikoagulans, dessen Wirkung auf der Bildung einer chemischen Verbindung mit Thrombin beruht, wodurch dessen katalytische Wirkung inhibiert wird. Hirudin ist ein Miniprotein aus 65 Aminosäuren mit einem Molekulargewicht von 7 kD. Aufgrund seiner starken Affinität zu Thrombin (k₁-Werte von > 10⁻¹² Mol/l) und seines direkten Wirkungsmechanismus ist es von großem Interesse. Seine klinische Anwendung war in der Vergangenheit äußerst beschränkt, da Hirudin nicht leicht in standardisierter Form zugänglich war und kein Gegenmittel verfügbar war. Hirudin kann heute naturidentisch gentechnologisch hergestellt werden, und daher wäre seine klinische Anwendung in der nächsten Zeit möglich.

Beispielsweise werden in der EP-A-0 468 327 pharmazeutische Präparate für die orale Verabreichung beschrieben, die rekombinantes Hirudin enthalten.

In letzter Zeit haben synthetische Thrombininhibitoren große Bedeutung erlangt. Augenblicklich wird weltweit in vielen Forschungslaboratorien zu derartigen synthetischen Inhibitoren Synthesearbeit geleistet. Am weitesten fortgeschritten sind die Untersuchungen mit Derivaten des Benzamidins, wie z.B. dem kleinmolekularen synthetischen Thrombininhibitor NAPAP (Nα-(2-Naphthylsulforylglycyl)-D,L-amidinophenylalaninpiperidid) und mit sogenannten Tripeptiden. Alle synthetischen Thrombininhibitoren befinden sich augenblicklich in der präklinischen Untersuchung. Ihre Wirkungen sind qualitativ denen von Hirudin gleichzusetzen. Der Metabolismus der synthetischen Thrombininhibitoren unterscheidet sich jedoch von dem des Hirudins. In der Regel werden die Thrombininhibitoren in der Leber oder im Blut metabolisiert. Es ist abzusehen, daß in Kürze derartige Substanzen für die klinische Erprobung zur Verfügung stehen. Der Vorteil gegenüber dem Hirudin liegt vor allem darin, daß diese Substanzen oral verabreicht werden können.

Trotz der günstigen Wirkungen ist der klinische Einsatz von Hirudin bis jetzt unterblieben bzw. erfolgt nur sehr eingeschränkt, da, wie oben erwähnt, kein Antidot zur Verfügung steht, welches z.B. beim Heparin mit dem Protaminsulfat gegeben ist. Das Vorhandensein eines Antidots ist bei einem derartig hochwirksamen Thrombininhibitor zwingend erforderlich, denn bei zufälligen Überdosierungen oder bei Patienten mit Nierenfunktionsstörungen, bei denen eine Blutungskomplikation droht, muß sofort ein Antidot vorhanden sein, wenn eine Überdosierung festgestellt wird. Derartige Blutungskomplikationen treten beispielsweise als hämorrhagische Nebenwirkungen auf, vor allem in den Gefäßgebieten des Peritoneums, der Pleura, des Pericards und der Pia mater, aber auch in Operationsschnittwunden, wie dies an Tieren mit extrem hohen Blutspiegeln eines Thrombininhibitors beobachtet wurde.

In der Vergangenheit wurden verschiedene Antidotprinzipien für Hirudin experimentell untersucht, so der Einsatz von Gammathrombinpräparaten, wie DFP-Thrombin oder Benzoylthrombin (Brüggener, E., Walsmann, P., Markwardt, F.: Neutralization of hirudin anticoagulant action by DIP-thrombin. Pharmazie 1989; 44: 648-9). Diese Präparate haben sich jedoch in der Praxis nicht bewährt, da ihre Affinität zu Hirudin zu gering ist und sie zu toxisch sind. Auch aktivierte Plasmafraktionen, wie FEIBA oder Autoplex-Präparate, sind wegen der thromboplastinähnlichen Aktivität ungeeignet (Fareed, J., Walenga, J.M.: Do we need to neutralize hirudin's anticoagulant effects to minimized bleeding. Fed Proc 1989; 3: A328; Walenga, J.M., Piffarre, R., Hoppensteadt, D.A., Fareed: Development of recombinant hirudin as a therapeutic anticoagulant and antithrombotic agent. Some objective considerations. Sem Thromb Hemost 1989; 15: 316-33).

Die Herstellung von humanen Blutplasmaprodukten erfolgt augenblicklich in Deutschland und weltweit generell aus zwei Quellen. Zunächst produzierten die Blutspende-Institute Blutkonserven und davon abgeleitete Plasmafraktionen oder Faktoren-angereicherte Plasmakomponenten (Kryopräzipitat, PPSB-Fraktion, Blutplättenkonzentrate, Erythrozytenkonzentrate u.a.). Das von Spendern abgenommene Blut wird nicht ausschließlich als Vollkonserve durch Kühlung für längere Zeit aufbewahrt, sondern man suchte Wege, um auch universellere und besser verwendbare Blutkomponenten herzustellen.

Ein weiterer großer Teil von Blutprodukten und Fraktionen wird durch kommerzielle Anbieter erzeugt, die fabrikmäßig sehr große Blut- und Blutplasma-Chargen aufkaufen und diese zu arzneimittelähnlichen Blutprodukten verarbeiten, die im wesentlichen den Prüfbedingungen des Arzneimittelgesetzes standhalten müssen (Antithrombin III, Faktor VIII, Faktor IX, Protein C, Faktorenkomplexe, Fibrinogen, Plasminogen u.a.).

Beiden Herstellungsverfahren liegt neuerdings die schwierige Aufgabe zugrunde, den Nachweis der Virusfreiheit zu erbringen. Sowohl Hepatitis B/C als auch Aids u.a. virale Erkrankungen haben es mit sich gebracht, daß die Blutprodukte des Menschen ganz neuen und viel strengeren Prüfvorschriften unterliegen.

Die Hersteller verwenden eine Reihe von Methoden, um das Blut nach dem Verlassen des Körpers als nicht mehr infektiös anzusehen. Typisches Beispiel ist die Methylenblau-Inaktivierung von eventuellen Virusverunreinigungen (HIV-Viren, Hepatitis B-Viren u.a.). Weitere Methoden sind die Delipidierung der Plasmafraktionen oder Wärmebehandlung, um eine entsprechende Abreicherung von Viren zu erreichen. Alle diese Präparate werden in dieser Hinsicht sehr streng geprüft, und die in den Verkehr gebrachten Präparate sind nach Stand der heutigen Technik als unbedenklich einzuschätzen.

Prothrombin oder daraus gewonnene Produkte werden nach Wissen der Erfinder bisher nicht als Blutfolgeprodukte klinisch eingesetzt. Die Reinigungsverfahren sind daher meist dem Forschungsbereich angepaßt. Die einzige bekannte publizierte Methode der Reinigung von humanem Prothrombin aus PPSB ist in Sakuragawa, N. et al., Acta Medica et Biologica 25 (2): 119-124, (1977), zu finden. Folgende Reinigungsmethoden für humanes Prothrombin sind bekannt, wobei hier ein virusfreies Produkt nicht gegeben ist:

Eine Fällung der Vitamin-K-abhängigen Faktoren erfolgt im Citrat-Plasma, im Kryopräzipitat, in Cohn-Fraktionen und ähnlichem mit Bariumchlorid (K.G. Mann, Methods in Enzymology, XLV, 123ff, 1976). Für eine klinische Anwendung müßte ein Nachweis geliefert werden, daß keine toxischen Bariumionen mehr im Produkt zu finden sind. Der Niederschlag wird in der Folge mit EDTA gelöst, umgepuffert, gefolgt von einer schrittweisen Fällung der noch vorhandenen Proteine mit Polyethylenglykol oder durch Aussalzen mit Ammoniumsulfat, dann einer Trennung der Prothrombin enthaltenden Fällungsniederschläge über eine Anionenaustauschersäule (DEAE, Diethylaminoethyl als funktionelle Gruppe wird vor allem auch im Produktionsmaßstab eingesetzt) sowie einer Trennung mittels Gelfiltration.

Eine andere mögliche Methode ist das Binden des Plasmas oder der oben genannten Fällungsprodukte zum Beispiel an eine Anionenaustauschermatrix wie DEAE, gefolgt von einem Waschen der Matrix und einer Elution der Faktoren mit einem NaCl-Gradienten in Puffer. Die Herstellung des Prothrombin-Komplex-Konzentrats erfolgt zumeist in dieser Weise durch Bindung des Kryopräzipitat-Überstandes und darauffolgender Elution mit 2 M Natriumchloridlösung in Puffer (H.G.J. Brummelhuis, in "Methods of Plasma Protein Fractionation", Hrsg. J.M. Curling; Academic Press 1980; Feldman, P. und Winkelman L., in "Blood Separation and Plasma Fractionation", Wiley-Liss, Inc.: 341-83, (1991)). Verwendet wird hier ein physiologisch verträglicher Tri-Natrium-Citrat-Puffer im neutralen pH-Wert-Bereich.

Weitere Reinigungsmethoden bestehen in der Bindung der prothrombinhaltigen Lösungen (aus Fällungen, Chromatographie-Elutionsfraktionen etc.) an eine Affinitätssäule mit einer Dextran-Sulfat-Matrix (Rosenberg, J.S. et al., J. Biol. Chem. 259: 1607-17, (1975); Miletich et al., J. Biol. Chem. 253: 6908-19, (1978)), einer Arginin-Matrix (Sakuragawa, N. et al., Acta Medica et Biologica 25 (2): 119-124, (1977)), einer Heparin-Matrix (Bajaj, S.P. et al. Preparative Biochemistry. 11(4): 397-412, (1981) oder an Matrices mit immobilisierten Anti-Prothrombin-Antikörpern. Die Methode mit immobilisierten Antikörpern ist jedoch sehr material- und kostenaufwendig. Für den nichtrekombinanten Faktor VIII wird diese Methode zum Beispiel eingesetzt, da der Faktor im Plasma in deutlich geringeren Konzentrationen vorhanden ist als zum Beispiel das Prothrombin und so stark angereichert werden kann.

Reekers and Hemker, Haemostasis, Bd. 1, Seiten 2-22, 1972 beschreiben ein Verfahren zur Reinigung der bovinen Blutgerinnungsfaktoren II, VII, IX und X aus einer als PPSB-2 bezeichneten Präparation. Die Reinigungsschritte umfassen Anionenaustauschchromatographie und Gelfiltration.

Die WO-A-93/15757 beschreibt ein Antidot für Hirudin, welches eine Verbindung, die Prothrombin zu Meizothrombin spaltet (Ecarin), ein Prothrombinintermediat, z.B. Meizothrombin-des-Fragement-1, ein pharmakologisch annehmbares Salz davon oder ein Gemisch dieser Verbindungen enthält.

Die DE-A-44 30 204 beschreibt ein Arzneimittel enthaltend Prothrombin zur Antagonisierung von Blutantikoagulantien.

Die genannten Methoden sind jedoch meist zeitaufwendig, mit der Verwendung toxischer Salze verbunden und führen nicht zu einem reinen virusfreien Endprodukt. Im übrigen werden Reinigungsschritte unter Verwendung toxischer Substanzen in der Regel von den Zulassungsbehörden abgelehnt. Somit besteht ein großer Bedarf nach einem Herstellungsverfahren für eine Antidotausgangssubstanz bzw. ein spezifisches Antidot für natürliche und synthetische Thrombininhibitoren.

Der vorliegenden Erfindung liegt daher die Aufgabe zugrunde, ein Verfahren zur Herstellung eines möglichst virusfreien Ausgangsmaterials für ein Antidot für natürliche oder synthetischen Thrombininhibitoren zur Verfügung zu stellen. Bei dem Verfahren sollen nur Arbeitsschritte verwendet werden, bei denen physiologisch unbedenkliche Substanzen eingesetzt werden. Eine Verunreinigung durch toxikologisch bedenkliche Verfahrensprodukte bzw. Beimischungen soll ausgeschlossen sein. Ferner soll die Herstellung im Sinne eines Blutfolgeprodukts, nicht eines der Arzneimittelgesetzgebung unterworfenen Arzneimittels erfolgen. Die Entwicklungszeit bis zur klinischen Einführung soll auf wenige Monate beschränkt sein. Das Verfahren soll einfach und kostengünstig durchzuführen sein.

Gegenstand der Erfindung ist ein Verfahren zur Herstellung einer hochgereinigten virusfreien Antidotausgangssubstanz für natürliche und synthetische Thrombininhibitoren, das dadurch gekennzeichnet ist, daß man ein Blutfolgeprodukt zuerst über eine Anionenaustauschersäule chromatographiert, dann die die Antidotausgangssubstanz enthaltende Fraktion einer Gelfiltration unterwirft, die reine Antidotausgangssubstanz isoliert und diese weiterhin einer Affinitätschromatographie mit einer Säule mit festgebundenem Schlangengiftenzym, ausgewählt aus Ecarin oder einem Enzym mit gleicher Wirkung, unterwirft, wobei ein reines Prothrombin-Thrombin-Aktivierungsintermediat erhalten wird.

Gegenstand der Erfindung ist weiterhin die Verwendung eines so erhaltenen Produkts zur Herstellung eines Antidots für einen natürlichen oder synthetischen Thrombininhibitor.

Die Erfinder haben überraschenderweise gefunden, daß, wenn man ein als Arzneimittel zugelassenes Blutfolgeprodukt, das von Blutspendediensten in ihren präparativen Abteilungen hergestellt wird, mit einem zweistufigen FPLC-Chromatographieverfahren behandelt, eine hochreine virusfreie Antidotausgangssubstanz erhalten werden kann. Bevorzugt werden Prothrombin und Präthrombin-1 erhalten.

Das Prothrombin liegt in solchen Präparaten in blutidentischer Form vor.

Das Präthrombin-1 ist ein stabiles konservatives Aktivierungsprodukt der Prothrombin-Thrombin-Aktivierungskaskade. Aus Präthrombin-1 kann beispielsweise hochreines α-Thrombin, aber auch Meizothrombin-des-Fragment-1 hergestellt werden.

Als Ausgangsmaterialien können beliebige Blutfolgeprodukte verwendet werden, die Prothrombin und/oder Präthrombin-1 enthalten und virusfrei sind, z.B. konzentrierte Vitamin-K-abhängige Gerinnungsfaktoren, virusfreie Plasmen oder Plasmafraktionen nach vorheriger Abtrennung von Plasmaproteinen. Bevorzugt wird die marktübliche PPSB-Fraktion verwendet, ein Multifaktoren-Komplex, der in der klinischen Medizin zur Substitution von Gerinnungsfaktormangel der Faktoren II, VII, IX und X, vor allem bei Verbrauchsreaktionen und schweren intensivmedizinischen Krankheitszuständen, benützt wird. Die vier Vitamin-K-abhängigen Faktoren, die die Hauptbestandteile von PPSB-Konzentraten ausmachen (PPSB: Prothrombin + Prokonvertin + Stewart-Prower-Faktor + antihämophiles Globulin B, entsprechend Faktor II, VII, IX und X), besitzen alle mehr oder weniger die gleichen physikalisch-chemischen Eigenschaften, wie Molekulargewicht (50 bis 70 kD), isoelektrischen Punkt pI und elektrophoretische Mobilität. Gerade der niedrige pI-Wert von 4,1 bis 4,6 ist für diese vier Faktoren charakteristisch. Die geringen Unterschiede des pI-Werts der einzelnen Faktoren erschweren eine chromatographische Trennung. Es wurde nun überraschenderweise gefunden, daß die Antidotausgangssubstanz aus diesem lyophilisierten Blutfolgeprodukt durch zwei chromatographische Schritte gereinigt werden kann. Dabei wird das Ausgangsmaterial zuerst über eine Anionenaustauschersäule chromatographiert und dann einer Gelfiltration unterworfen.

Es können beliebige Kombinationen eines Anionenaustauschers mit einer Gelfiltration verwendet werden, wobei die Elution mit einem wäßrigen Puffersystem mit einem Gradienten aus einem physiologisch verträglichen Salz durchgeführt wird. Die chromatographische Trennung wird in einem geeigneten System vorgenommen. Bevorzugt hierzu ist das FPLC®-System anzunehmen.

Bei dem Anionenaustauscherharz handelt es sich bevorzugt um die ResourceQ®, 6-ml-Säule der Firma Pharmacia, Uppsala, Schweden. Das Säulenmaterial ist ein starker Anionenaustauscher (SourceQ®15) mit monodispersem 15-µm-Material aus Polystyrol/Divinylbenzol. Die aktiven Gruppen bestehen aus quarternärem Ammonium. Die typischen Flußraten sind 1 bis 10 ml/min bei einem maximalen Gegendruck von 4 MPa (40 bar, 600 psi). Die pH-Stabilität liegt bei pH 2 bis 14 im Arbeitsbereich (Herstellerangaben). Als alternative Matrices für die Ionenaustauschchromatographie bieten sich jedoch auch Matrices mit anderen funktionellen Gruppen an. Beispiele hierfür sind DEAE (Diethylaminoethyl), QAE (quarternäres Aminoethyl) oder quarternäres Ammonium.

Als Puffersystem wurde ein 25 mM Tri-Natrium-Citrat-Puffer, pH 7,0 mit HCl eingestellt, sowie zur Elution der obige Puffer inklusive 1 Mol/l Natriumchlorid gewählt, um einen physiologisch verträglichen Puffer zu verwenden und ein Umpuffern zu umgehen. Für die eigentliche Trennung sind prinzipiell ähnliche Puffersysteme mit gleichem pH-Bereich und ähnlicher Ionenstärke verwendbar, wie Tris-HCl-Puffer und andere. Der Vorteil eines physiologisch verträglichen Puffers wäre hier nicht gegeben und ein Umpuffern notwendig.

Prinzipiell sind für die Reinigung des Prothrombins durch die Anionenaustauschchromatographie alle gängigen Puffersysteme verwendbar. Vorteilhaft für die spätere klinische Anwendung des Produktes sind neben den geringen Kosten die Verwendung eines physiologisch verträglichen Puffers wie der benutzte Tri-Natrium-Citrat-Puffer. Der verwendete pH-Wert und die eingestellte Ionenstärke müssen der Stabilität des Prothrombins und den chromatographischen Eigenschaften des verwendeten Materials Rechnung tragen. Weitere Puffersysteme sind zum Beispiel MES (2-(n-Morpholino)ethansulfonsäure)-Puffer, Bis-Tris ((Bis(2-hydroxyethyl)imino)tris(hydroxymethyl)methan)-Puffer, PIPES (Piperazin-N,N'-bis(2-ethansulfonsäure))-Puffer, MOPSO (3-(N-Morpholino)-2-hydroxypropansulfonsäure)-Puffer, Imidazol-Puffer, MOPS (3-(N-Morpholino)propansulfonsäure)-Puffer, weitere Good-Puffer und verwandte Puffersysteme.

Das Gelfiltrationsmaterial ist so zu wählen, daß eine möglichst optimale Trennung der großmolekularen Verunreinigungen nach der Anionenaustauschchromatographie und dem Produkt, z.B. Prothrombin oder Präthrombin-1, gewährleistet ist. Jedes Gelfiltrationsmaterial, das diese Kriterien erfüllt, ist im Prinzip für dieses Verfahren anwendbar. Geeignet sind alle gängigen Gelmatrizes, wie z.B von den Firmen Pharmacia (Sephacryl® Superose®, Superdex®), Trisacryl, die Materialien von Merck/Toyo Soda (TSK®, Toyopearl®), Amicon (Matrex Cellufine®), BioRad (BioGel®), und andere, die eine Trennung des Prothrombins mit einem Molekulargewicht von etwa 72 Kilodalton von verschiedenen Verunreinigungen und möglichen Abbauprodukten des Prothrombins zulassen, die ein Molekulargewicht größer beziehungsweise kleiner 70 Kilodalton besitzen. Erfahrungsgemäß sind bei den PPSB-Fraktionen hierfür Gelfiltrationsmatrizes mit einem Fraktionierungsbereich zwischen minimal 1 Kilodalton und maximal 1500 Kilodalton ausreichend (bevorzugt 5 kD-250 kD). Bei anderen Ausgangsmaterialien für die Prothrombin-Isolierung könnten Fraktionierungsbereiche oberhalb der angegebenen nötig sein, die aber von einem Fachmann leicht durch Routineversuche ermittelt werden können. Für das hier gewählte Puffersystem gelten die gleichen Ausführungen wie für die Ionenaustauschchromatographie.

Die beigefügten Figuren erläutern die Erfindung näher; darin sind:

### Figur 1:

Bearbeitetes Elektropherogramm (SDS-PAGE) einer Probe des Ausgangsmaterials PPSB, Coomassie gefärbt.

### Figur 2:

Chromatogramm der Trennung einer PPSB-Probe über die Anionenaustauschersäule ResourceQ®. Dargestellt sind die UV-Absorption in Absorption Units (AU) sowie der Elutionsgradient in Prozent Puffer B gegen die ml Elutionslösung. a) Ausdruck vom FPLC-Assistant® (Auswertesoftware der FPLC), b) bearbeitete Daten in NeXT®. Darüberprojeziert sind die Ausschnitte der Elektropherogramme der einzelnen analysierten Fraktionen.

### Figur 3:

Elektropherogramm (SDS-PAGE) des Vergleichs zweier kommerziell bezogener humaner Prothrombin-Proben und des aus PPSB gewonnenen Prothrombins.

### Figur 4:

Chromatogramm und Elektropherogramm der Fraktionen einer Trennung des Materials nach der Anionenaustauschersäule durch die Gelfiltration. a) Ausdruck vom FPLC-Assistant®, b) bearbeitete Daten in NeXT®. Darüberprojeziert ist der Ausschnitt des Elektropherogramms der einzelnen analysierten Fraktionen. Das isolierte Prothrombin ist auch nach der Analyse mittels SDS-PAGE und Silberfärbung als zu 95 % bis 99 % rein zu bezeichnen.

### Figur 5:

Analyse einer mit BaCl₂ gefällten und über DEAE-Sephacel getrennten PPSB-Probe

### Figur 6:

Auftrennung einer PPSB-Probe über ResourceQ®

### Figur 7:

Elektropherogramm der Herstellung von humanem Meizothrombin-des-Fragment-1 aus humanem Prothrombin, welches aus PPSB gewonnen wurde, mit immobilisiertem Ecarin.

Durch diese chromatographischen Verfahren können reines Prothrombin und reines Präthrombin-1 erhalten werden. Die Aktivierung des gereinigten Prothrombins oder Präthrombin-1 mit Ecarin, dem Prothrombin-Aktivator aus dem Schlangengift von Echis carinatus, oder einem Schlangengiftenzym mit gleicher Wirkungsrichtung, beispielsweise aus Bothrops neuwiedi, Dispholidus typus, Notechis scutatus, Bothrops atrox Maranhao, Bothrops atrox Moojeni, Oxyuranus scutellatus, liefert die bekannten Prothrombin-Thrombin-Aktivierungsintermediate Meizothrombin und Meizothrombin-des-Fragment-1. Daraus kann weiter α-Thrombin hergestellt werden.

Die folgenden Beispiele erläutern das erfindungsgemäße Verfahren näher.

### Beispiel 1

### Isolierung von humanem Prothrombin aus PPSB-Präparaten über Anionenaustauschchromatographie und Gelfiltration (Verwendung einer ResourceQ®-Säule)

### 1. Material und Methode

### 1.1. Material

Prothrombin-Komplex-Konzentrate: PPSB-250-bulk: DRK-Blutspendedienst Niedersachsen, Springe; Ch.-B. 4920 NNN 656 (im folgenden PPSB). Ein Vial enthält laut Analyseprotokoll des Blutspendedienstes 200 mg Protein, davon Faktor II (Prothrombin) 25 mg (244 I.E.).

Die Chromatographie erfolgte am FPLC®-System (Pharmacia, Uppsala, Schweden). Die Anionenaustauschersäule ResourceQ®, 6-ml-Säule, die Gelfiltrationssäule Superdex 200, HR 10/30, und die Entsalzungssäulen PD10 wurden ebenfalls von Pharmacia bezogen. Alle Chemikalien wurden von Fluka-Chemie (Buchs, Schweiz) erhalten. Die Elektrophorese wurde an der Apparatur MiniProteanII mit der Spannungsquelle PowerPac 300 oder PowerPac 3000 von BioRad, München, vorgenommen. Die Aktivierung des Prothrombins wurde am Spektralphotometer Biochrom 4060 von Pharmacia verfolgt (siehe Aktivierungsassay). Zum Einengen größerer Probenvolumina wurde die Ultrafiltrationszelle (maximales Volumen 200 ml) von Amicon (Beverly, USA) verwendet und mit Stickstoff betrieben. Die Membran für die Ultrafiltration (Trenngrenze 10 kD) war die YM10-Membran Diaflo (Amicon). Zum Einscannen der SDS-PAGE-Gele wurde der Scanner HP ScanJet IIc von Hewlett Packard (Hewlett Packard GmbH, Waldbronn) eingesetzt.

### 1.2. Methode

### Proteinbestimmung:

Die Proteinbestimmung erfolgte nach der BCA-Methode (Smith et al, Anal. Biochem., 150, 76 (1985)) (Bicinchoninic-Acid) mit Reagentien und nach Vorschrift von Sigma (Sigma-Chemie, Deisenhofen).

### Chromatographie:

Ein Vial PPSB wurde in 10 ml steriles Wasser aufgelöst, durch einen 0,2-µm-Sterilfilter (Schleicher & Schuell, Kassel) gegeben, und 5 ml wurden mit einer Schlauchpumpe auf die ResourceQ-Säule gegeben. In 10 ml Lösung sind ca. 200 mg Protein und 25 mg Prothrombin enthalten.

Es wurde so lange mit Puffer A (Puffer A, 25 mM Tri-Natrium-Citrat-Puffer, pH 7,0; Puffer B, A + 1 M NaCl) gespült, bis die Absorption im UV-Detektor ein stabiles Minimum erreichte. Das Prothrombin wurde dann am FPLC®-System mit einem NaCl-Gradienten (Puffer B) eluiert (2 Säulenvolumina 0,1 % B, dann 6 Säulenvolumina eines linearen Gradienten von 20 % bis 40 % Puffer B). Das Prothrombin eluiert hierbei bei 30 % Puffer B. Die Prothrombin enthaltenden Fraktionen wurden mittels SDS-PAGE analysiert, gepoolt, und der Proteingehalt wurde bestimmt. Die Proteinkonzentration lag in den gepoolten Fraktionen bei 1,0 bis 1,5 mg/ml. Die gepoolten Fraktionen mehrerer Läufe wurden dann durch Ultrafiltration eingeengt, mit PD10-Säulen in 25 mM Tri-Natrium-Citrat-Puffer, 0,1 M NaCl, pH 7,0, umgepuffert und über Gelfiltration weiter gereinigt. Maximal 500 µl Probe wurden aufgegeben und mit einem maximalen Fluß von 0,5 ml/min getrennt. Die gesammelten Proben wurden ebenfalls durch Gelelektrophorese analysiert.

### SDS-PAGE:

### (SDS-PAGE:Sodiumdodecylsulfat-Polyacrylamidgelelektrophorese)

Es wurden Elektrophoresen mit 8 %, 10 %, 12 oder 13 %-Trenngele und 4 % Sammelgel sowie 4 bis 20 % Gradientengele mit dem Laemmli-Puffer-System (Laemmli, U.K., Nature, 227, 680 (1970)) gefahren. Die Elektropherogramme wurden mit Coomassie-BrilliantBlue R250-Lösung oder mit dem SilverStaining-Kit von Pharmacia angefärbt. Die Chromatographiefraktionen wurden über Gelelektrophorese auf die Qualität des isolierten Prothrombins analysiert. Je 10 bis 20 µl Probe wurden mit 60 µl Probenpuffer versetzt, und 25 µl wurden für die Elektrophorese eingesetzt. Als Standards wurden Phosphorylase b, Serumalbumin, Ovalbumin, Carboanhydrase, Trypsin-Inhibitor und Lysozym eingesetzt.

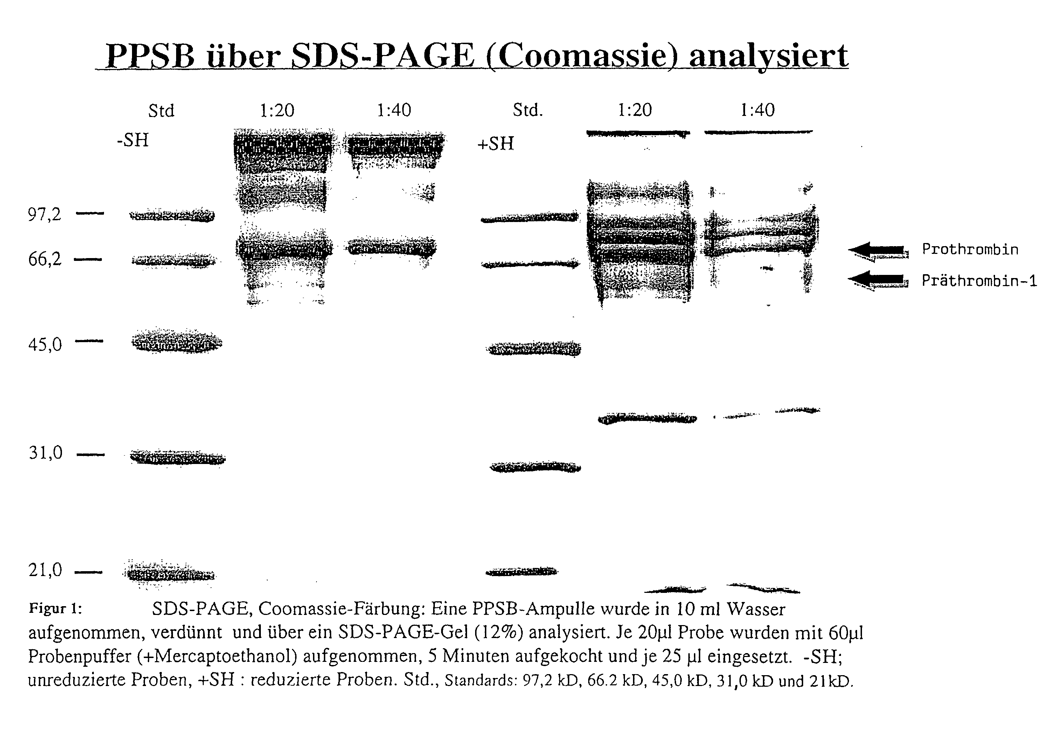

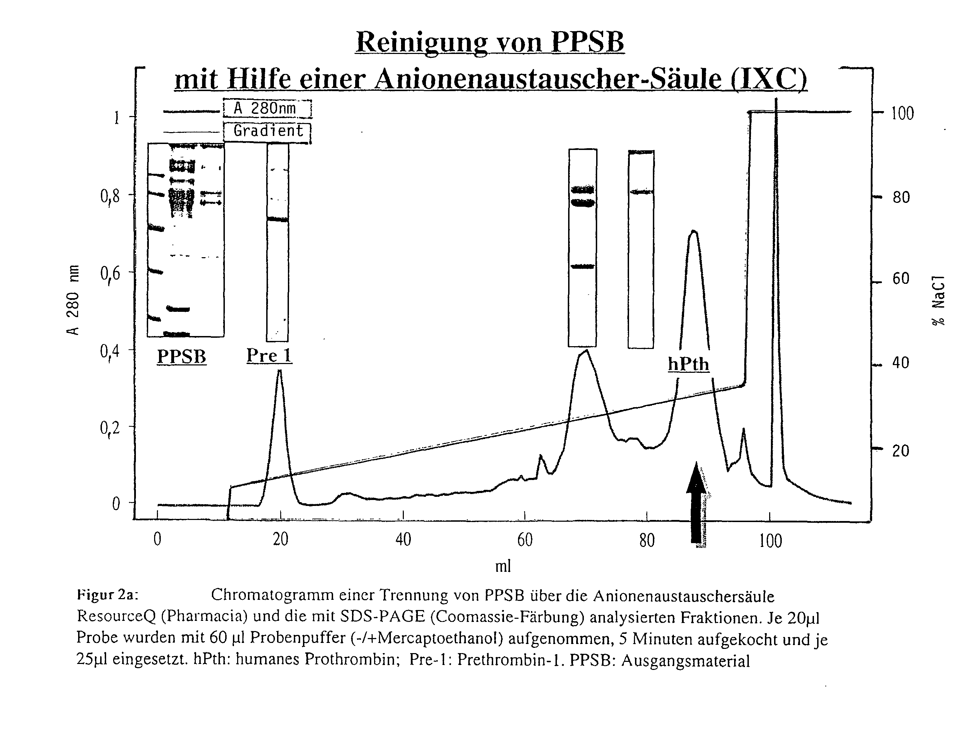

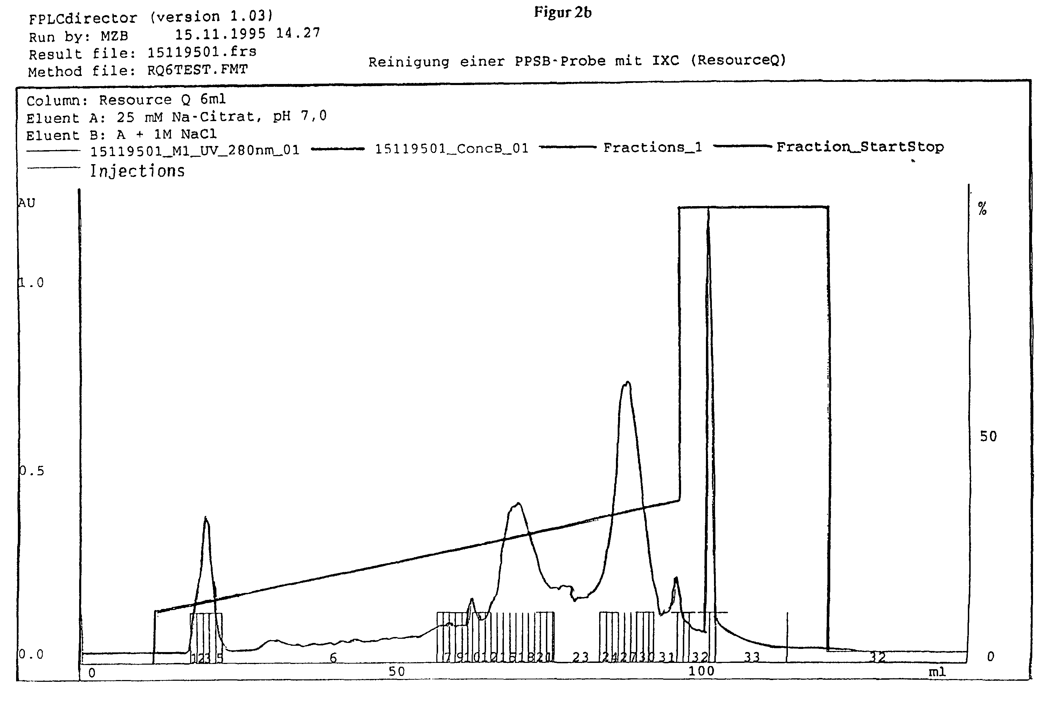

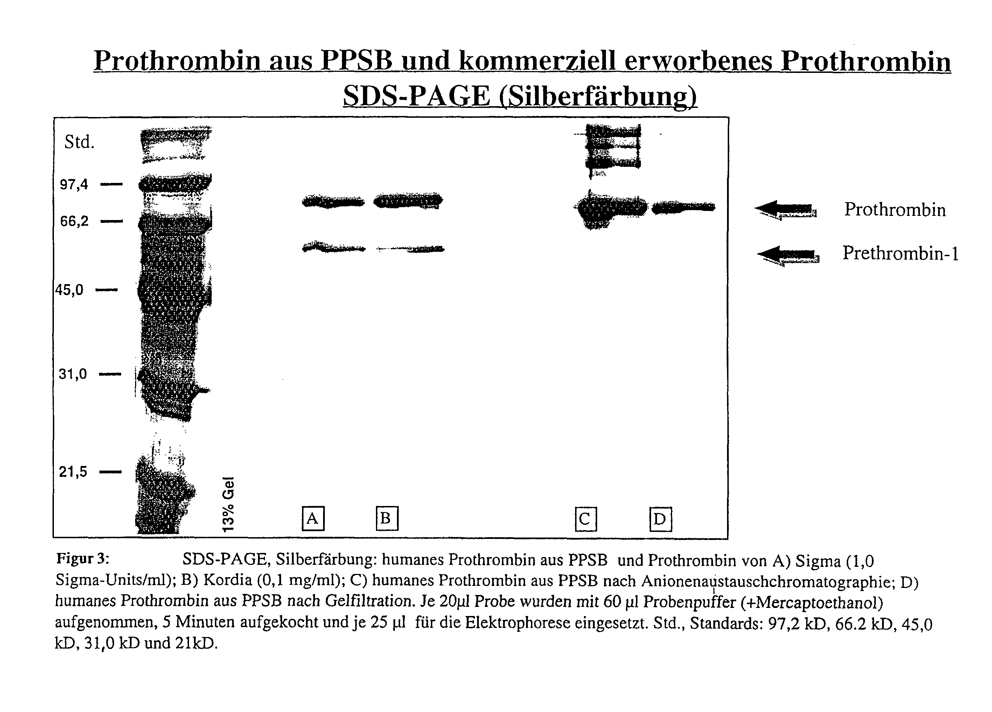

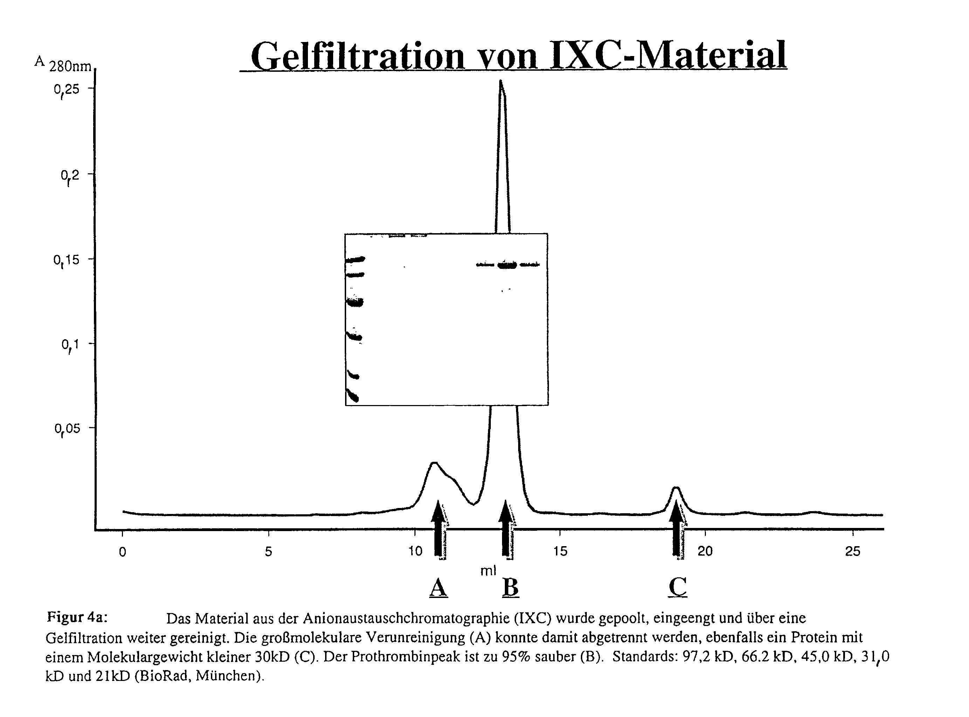

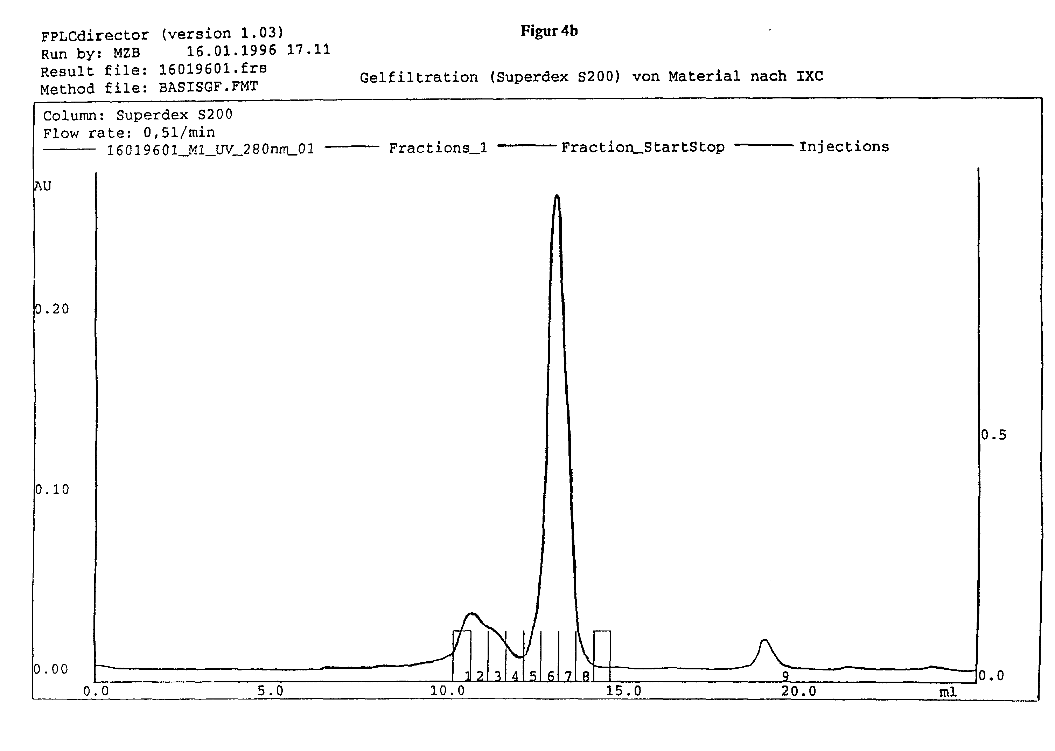

Die Ergebnisse sind in den Figuren 1 bis 4 gezeigt.

### Beispiel 2

### Isolierung von humanem Prothrombin und Präthrombin aus PPSB-Präparaten über Anionenaustauschchromatographie und Gelfiltration (Verwendung einer DEAE-Sephacel-Säule)

### 1. Material und Methode

### 1.1 Material

### PPSB-250-bulk: DRK-Blutspendedienst Niedersachsen, Springe, Ch.-B. 4920 NNN 656

DEAE-Sephacel-Säule: 1,5 x 20 cm

| **Lösungen**: | | |
|---|---|---|
| Tri-Natrium-Citrat | 294,1 g/Mol | Fluka |
| 3,6 % (0,122 M), Na-Citrat | 36 g/l | Laborchemie Apolda |
| 1 M BaCl₂ | 244,28 g/l | Laborchemie Apolda |
| 9 % NaCl- | 90 g/l | Fluka |
| 0,25 M Na-Citrat-Stock-Lsg. | 73,5 g/l | Laborchemie Apolda |
| 0,025 M Citrat-Puffer, pH 7,0 | | |
| 0,2 M EDTA pH 7,4 | 74,44 g/l | Sigma |
| (NH₄)₂SO₄ | | Fluka |
| 1 M Benzamidin-Hydrochlorid | 156,6 g/l | Sigma |
| (Sojabohnen-Trypsin-Inhibitor (SBTI) | (6000 BAEE units/mg) | Fluka) |
| Puffer A: 25 mM Natrium-Citrat-Puffer, pH 7,0 (294,1 g/Mol) | | |
| Puffer B: A + 1 M NaCl, 04.09.1995 | | |

### 1.2 Methode

### 25 mM Na-Citrat, 100 mM BACl₂

1 Flasche PPSB wurde in je 10 ml H₂O gelöst, mit 3,6 % Tri-Natrium-Citrat auf 25 mM gebracht und langsam mit 1 M BaCl₂ auf 100 mM Bariumchlorid eingestellt. Die Lösung wurde auf 1 mM Benzamidin und 10 mg/l SBTI gebracht und über Nacht gefällt. Bis zu 20 Flaschen PPSB-Konzentrat wurde in dieser Weise auf einmal aufgearbeitet. Der Überstand wurde mit Bariumchlorid und Tri-Natrium-Citrat gefällt wie oben, und dieser Schritt wurde ein weiteres Mal wiederholt. Das Pellet wurde dazwischen bei 3600 g abzentrifugiert und der Überstand verworfen. Der Niederschlag wurde mit 0,2 M EDTA (1/10 des Ausgangsvolumens) resuspendiert, und es wurde 1/10 Volumen 0,9 % NaCl, 25 mM Na-Citrat zugegeben. Es folgte eine fraktionierte Fällung mit (NH₄)₂SO₄ auf 10 % Ammoniumsulfat über Nacht. Anschließend wurde zentrifugiert, dann wurde der Überstand mit 40 % (NH₄)₂SO₄ gefällt. Es wurde erneut zentrifugiert, dann der Überstand aus 40 % (NH₄)₂SO₄ auf 70 % gebracht und für mindestens 3 Stunden gefällt. Der Niederschlag wurde in 25 mM Na-Citrat-Puffer, pH 7,0 aufgelöst und über Pharmacia PD-10-Säulen in Na-Citrat-Puffer umgepuffert.

Die umgepufferte Lösung aus der fraktionierten Ammoniumsulfatfällung wurde auf eine DEAE-Sephacel-Säule gegeben (1,5 x 20 cm). Puffer A: 25 mM Tri-Natrium-Citrat, pH 7,0; Puffer B: A + 1,0 M NaCl. Die Säule wurde mit einem Fluß von 0,5 ml/min äquilibriert. Die Probe wurde mit 0,3 ml/min über eine Probenschleife aufgegeben und Puffer A beibehalten, bis die Absorption ein Minimum erreicht hatte. Der Gradient wurde mit 0,25 ml/min von 0 bis 1 M NaCl in Puffer B gefahren.

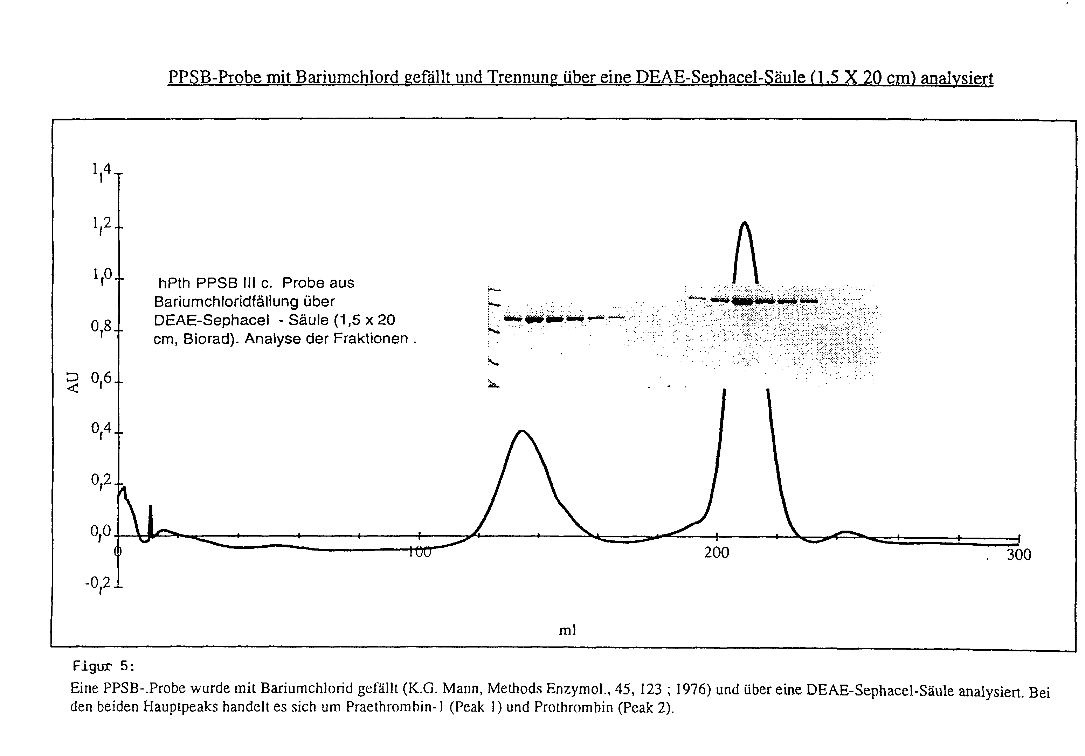

Das Ergebnis ist in Figur 5 gezeigt.

Die Fraktionen wurden mittels SDS-PAGE analysiert. Proben je 20 µl + 60 µl Probenpuffer wurden für die Elektrophorese vorbereitet und je 25 µl eingesetzt.

Die Qualität der analysierten Fraktionen war gut. Im ersten Peak befand sich überwiegend Präthrombin-1, im zweiten überwiegend Prothrombin.

### Beispiel 3

### Isolierung von humanem Präthrombin-1 aus PPSB-Präparaten mit Hilfe von Ionenaustauschchromatographie

### 1. Material und Methode

### 1.1 Material

### Prothrombin-Komplex-Konzentrate:

PPSB-250-bulk: DRK-Blutspendedienst Niedersachsen, Springe, Ch.-B. 4920 NNN 656. 1 Ampulle enthält gemäß Analysenprotokoll 200 mg Protein, enthaltend Faktor II (Prothrombin), 25 mg (244 I.E.).

1 Ampulle wurde mit 5 ml Chromatographie-Puffer gelöst und für den Gebrauch verdünnt. Die Chromatographie erfolgte mit Hilfe eines FPLC-Systems (Pharmacia, Uppsala, Schweden). Als Anionenaustauschersäule wurde eine ResourceQ, 6 ml-Säule verwendet. Die Entsalzungssäulen PD-10 wurden ebenfalls von Pharmacia bezogen. Alle verwendeten Chemikalien kamen von der Fluka-Chemie, Buchs, Schweiz. Zur Elektrophorese wurde die MiniProteanII-Apparatur mit der Spannungsquelle PowerPac 300 bzw. 3000 von BioRad, München verwendet. Zur Einengung größerer Probenvolumina wurde die Ultra-Filtrationszelle (200 ml) von Amicon, Beverly, USA verwendet und mit Stickstoff betrieben. Die Membran für die Ultra-Filtration (Trenngrenze KD) war die YM10-Membran Diaflo (Amicon). Die SDS-PAGE-Gele wurden mit dem Scanner HP ScanJet IIc von Hewlett Packard GmbH, Waldbronn eingescannt.

### 1.2. Methode

### Proteinbestimmung:

Die Proteinbestimmung erfolgte mit der BCA-Methode (Smith et al., Anal. Biochem., 150-76, 1985, Bicinchoninic-Acid) nach Originalvorschrift von Sigma-Chemie, Deisenhofen.

### Chromatographie:

1 Ampulle PPSB wurde mit 5 ml Chromatographie-Puffer aufgelöst und mit 5 ml sterilem Wasser verdünnt, danach durch einen 0,2 µm-Sterilfilter (Schleicher und Schüll, Kassel) gegeben. Die steril filtrierte Lösung wurde mit Hilfe einer Schlauchpumpe auf die ResourceQ-Säule gegeben. In der Lösung waren etwa 200 mg Protein enthalten. Die mit der PPSB-Fraktion beladene ResourceQ-Säule wurde mit Puffer A (Puffer A: 25 mM Tri-Natrium-Citrat-Puffer, pH 7,0; Puffer B: A + 1 M NaCl) gespült, bis die Absorption im UV-Detektor ein stabiles Minimum erreichte. Das Präthrombin-1 wurde von der ResourceQ-Säule mit einem NaCl-Gradienten (Puffer B) eluiert (2 Säulenvolumina 0,1 % B, dann 6 Säulenvolumina eines linearen Gradienten von 20 bis 40 % Puffer B). Das Präthrombin-1 eluiert hierbei bei etwa 20 % Puffer B. Die Präthrombin-1 enthaltende Fraktion wurde mittels SDS-PAGE analysiert, gesammelt, vereinigt und der Proteingehalt bestimmt.

Die Proteinkonzentration lag in den gepoolten Fraktionen bei 0,7 bis 1,0 mg/ml. Die Fraktionen wurden mit Hilfe der Ultrafiltration eingeengt, entsalzt, mit PD-10-Säulen in 25 mM Tri-Natrium-Citrat-Puffer, 0,1 M NaCl, pH 7,0 umgepuffert.

### SDS-PAGE:

Es wurden Elektrophoresen mit 8, 10, 12 oder 13 % Trenngel und 4 % Sammelgel sowie 4 bis 20 % Gradientengel mit dem Laemmli-Puffersystem gefahren, die Elektropherogramme wurden mit Coomassie-BrillantBlue R250-Lösung und mit Silver-Strain-Kit von Pharmacia gefärbt. Je 10 bis 20 µl Probe wurden mit 60 ml Probenpuffer versetzt, und 25 µl wurden für die Elektrophorese eingesetzt und mit bekannten Standards verglichen.

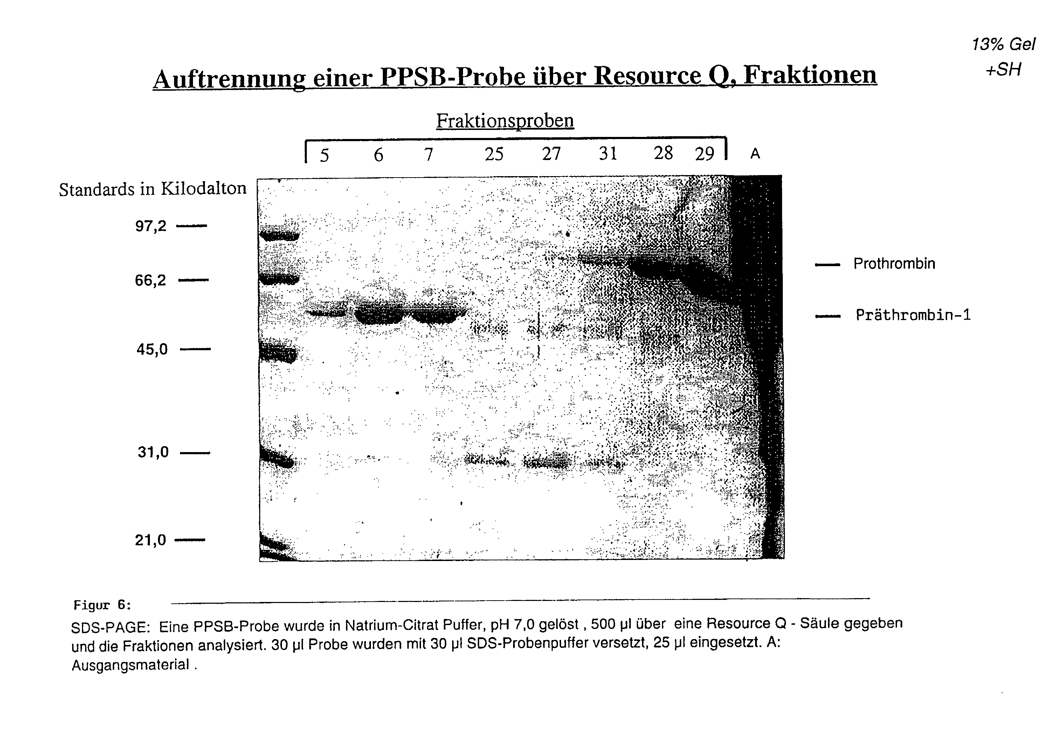

Das Ergebnis ist in Figur 6 gezeigt.

### Beispiel 4

### Aktivierung von gereinigtem Prothrombin bzw. Präthrombin-1 mit Ecarin

Zur Aktivierung der in den Beispielen 1, 2 bzw. 3 dargestellten reinen Prothrombin- bzw. Präthrombin-1-Präparationen wird ein Ecarin-Immobilisat der Fa. Pentapharm, Basel, Schweiz verwendet. Ecarin-Immobilisat-Lot: 6/116/03

Dieses Immobilisat ist problemlos handhabbar und weist eine sehr gute Stabilität auf. Nach Waschen des Immobilisats kann dieses über längere Zeit aufbewahrt werden. Das Ecarin-Immobilisat, vom Hersteller in flüssiger Form geliefert, wird in eine 6 ml-Säule gefüllt. Humanes Prothrombin aus PPSB (1 mg, 770 µl, 11/95) wird auf die Ecarinsäule gegeben und danach mit Tris-Puffer eluiert (50 mM Tris-HCl, 0,15 Mol NaCl, pH 8,0). Die Sammlung des Eluats erfolgt direkt im Chromatographie-Kühlschrank. Die Aktivierungssäule befindet sich bei Raumtemperatur neben dem gekühlten Fraktionssammler. Zur Aktivierung von humanem Präthrombin-1, welches aus der PPSB-Fraktion gewonnen wurde (0,8 mg, 760 µl, 3/96), wird in gleicher Weise wie für humanes Prothrombin beschrieben verfahren. Die erhaltenen Fraktionen lassen sich mit Hilfe der SDS-PAGE-Technik darstellen und analysieren. Es kann gezeigt werden, daß überwiegend oder nahezu ausschließlich Meizothrombin-des-Fragment-1 erhalten wird. Die Aktivierung erfolgt sowohl aus Prothrombin als auch aus Präthrombin-1 in gleicher Weise wie aus Ecarin. Zur Verhinderung einer Autoaktivierung werden den vereinigten Fraktionen 10 IU Heparin/ml zusammen mit 80 IU/ml Antithrombin III (human) gegeben. Der AT III-Gehalt ist zur Stabilisierung der Eiweißlösung ebenfalls notwendig.

Der Meizothrombin-des-F1-Gehalt der Lösung wird mit chromogenem Substrat S 2238/Chromogenix, Mölndal, Schweden bestimmt und kalibriert.

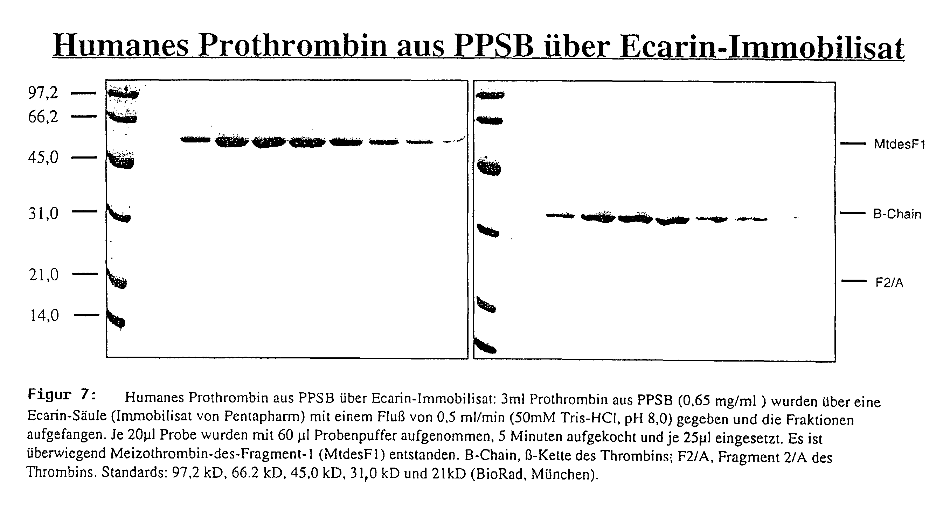

Die Ergebnisse sind in Figur 7 gezeigt.

## Patentansprüche

1. Verfahren zur Herstellung einer hochgereinigten virusfreien Antidotausgangssubstanz für natürliche und synthetische Thrombininhibitoren, **dadurch gekennzeichnet, daß** man ein Blutfolgeprodukt zuerst über eine Anionenaustauschersäule chromatographiert, dann die die Antidotausgangssubstanz enthaltende Fraktion einer Gelfiltration unterwirft, die reine Antidotausgangssubstanz isoliert und diese weiterhin einer Affinitätschromatographie mit einer Säule mit festgebundenem Schlangengiftenzym, ausgewählt aus Ecarin oder einem Enzym mit gleicher Wirkung, unterwirft, wobei ein reines Prothrombin-Thrombin-Aktivierungsintermediat erhalten wird.

2. Verfahren nach Anspruch 1, dadurch daß die isolierte Antidotausgangssubstanz Prothrombin ist.

3. Verfahren nach mindestens einem der Ansprüche 1 bis 2, **dadurch gekennzeichnet, daß** die isolierte Antidotausgangssubstanz Präthrombin-1 ist.

4. Verfahren nach mindestens einem der Ansprüche 1 bis 3, **dadurch gekennzeichnet, daß** das Blutfolgeprodukt eine handelsübliche PPSB-Fraktion ist.

5. Verfahren nach mindestens einem der Ansprüche 1 bis 4, **dadurch gekennzeichnet, daß** man die Chromatographie über die Anionenaustauschersäule an einer Säule mit einem starken Anionenaustauscher mit einem wäßrigen Puffersystem mit einem Gradienten aus einem physiologisch verträglichen Salz durchführt.

6. Verfahren nach mindestens einem der Ansprüche 1 bis 5, **dadurch gekennzeichnet, daß** man die Gelfiltration mit Gelfiltrationsmatrizes mit einem Fraktionierungsbereich von 1 kD bis 1500 kD und einem physiologisch verträglichen Puffer durchführt.

7. Verwendung des nach Anspruch 3 erhaltenen Produkts zur Herstellung eines Antidots für einen natürlichen oder synthetischen Thrombininhibitor.

## Claims

1. Process for producing a highly purified, virus-free antidote starting substance for natural and synthetic thrombin inhibitors, **characterised in that** a blood extract product is first of all chromatographed using an anion exchange column, the fraction containing the antidote starting substance is then subjected to gel filtration, the pure antidote starting substance is isolated and further subjected to affinity chromatography in a column containing bound snake venom enzyme selected from ecarin or an enzyme having the same effect, a pure prothrombin-thrombin activation intermediate thereby being obtained.

2. Process according to at least one of claims 1, **characterised in that** the isolated antidote starting substance is prothrombin.

3. Process according to at least one of claims 1 to 2, **characterised in that** the isolated antidote starting substance is prethrombin-1.

4. Process according to at least one of claims 1 to 3, **characterised in that** the blood extract product is a commercially available PPSB fraction.

5. Process according to at least one of claims 1 to 4, **characterised in that** the chromatography using the anion exchange column is carried out in a column containing a strong anion exchanger with an aqueous buffer system with a gradient of a physiologically compatible salt.

6. Process according to at least one of claims 1 to 5, **characterised in that** the gel filtration is performed with gel filtration matrices having a fractionation range from 1 kD to 1500 kD and a physiologically compatible buffer.

7. Use of a product obtained according to claim 3 for producing an antidote for a natural or synthetic thrombin inhibitor.

## Revendications

1. Procédé de préparation d'une substance antidote de base, exempte de virus et hautement purifiée, pour des inhibiteurs naturels et synthétiques de thrombine, **caractérisé en ce que** l'on chromatographie d'abord un dérivé sanguin sur une colonne échangeuse d'anions, que l'on soumet ensuite la fraction contenant la substance antidote de départ à une filtration sur gel qui isole la substance antidote de départ pure et soumet encore celle-ci à une chromatographie d'affinité avec une colonne ayant une enzyme de venins de serpent solidement liée, choisie parmi l'écarine ou une enzyme de même effet, un intermédiaire d'activation pur de la prothrombine et de la thrombine étant obtenu.

2. Procédé selon la revendication 1, **caractérisé en ce que** la substance antidote de départ isolée est la prothrombine.

3. Procédé selon au moins une des revendications 1 à 2, **caractérisé en ce que** la substance antidote de départ est la préthrombine-1.

4. Procédé selon au moins une des revendications 1 à 3, **caractérisé en ce que** le dérivé sanguin est une fraction PPSB usuelle dans le commerce.

5. Procédé selon au moins une des revendications 1 à 4, **caractérisé en ce que** l'on réalise la chromatographie sur colonne échangeuse d'anions sur une colonne ayant un échangeur d'anions fort avec un système tampon aqueux avec un gradient à partir d'un sel physiologiquement acceptable.

6. Procédé selon au moins l'une des revendications 1 à 5, **caractérisé en ce que** l'on réalise la filtration sur gel avec des matrices de filtration sur gel ayant une plage de fractionnement allant de 1 kD à 1500 kD et un tampon physiologiquement acceptable.

7. Utilisation du produit obtenu selon la revendication 3 pour la préparation d'un antidote pour un inhibiteur de thrombine naturel ou synthétique.
